(19) Europäisches Patentamt European Patent Office Office européen des brevets

(11) **EP 4 749 635 A1**

(12) **EUROPEAN PATENT APPLICATION**

(43) Date of publication:
**27.05.2026 Bulletin 2026/22**

(51) International Patent Classification (IPC):
**G16H 20/10** (2018.01)

(21) Application number: **24214924.3**

(22) Date of filing: **22.11.2024**

(52) Cooperative Patent Classification (CPC):
**G16H 20/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Health-AI Holdings Limited Ipswich IP3 9NP (GB)**

(72) Inventor: **DEWHURST, Robert Leslie Ipswich, IP3 9N (GB)**

(74) Representative: **Marks & Clerk LLP 15 Fetter Lane London EC4A 1BW (GB)**

(54) **COMPUTER SYSTEM FOR MANAGING DOSAGE TIMING OF TACROLIMUS**

(57) A data processing system configured to provide to a user, an indication of an optimal timing for regular administration of tacrolimus to a patient and provide to the user, an indication of an acceptable range around the optimal timing at which a dose of tacrolimus may be taken. The Tac levels are predicted based upon a previous measurement of the Tac concentration in serum of the patient and based upon the current dosage and body weight of the patient. This predication is then used, along with one or more earlier measurement, to establish a baseline concentration value against which deviations can be measured. If a new measurement indicates a significant deviation from the baseline concentration value, where this deviation is above a threshold, the acceptable range is reduced an alert is transmitted. In this way, greater timing discipline is enforced if large deviations from the baseline are measured.

Figure 8

**Description**

Technical Field

The present disclosure relates to a computer system for managing dosage timing of Tacrolimus and, in particular, to a computer system for indicating of a timing range for a dosage of Tacrolimus.

Background

Transplant rejection may occur following the transplantation of an organ from one person to another. When such rejection occurs, the body's immune system attacks the transplanted organ in response to recognising the organ as originating from a different person. The probability of rejection of a transplanted kidney in the first year following the kidney transplant is between 10% and 15%.

Tacrolimus is an immunosuppressive drug, which serves to lower the probability of organ rejection. Following the organ transplant operation, tacrolimus should be taken over the long term in order to lower the probability of organ rejection. Tacrolimus has a narrow therapeutic time range and doses are taken every 12 hours. Hence, it is required to carefully control dose timing by a patient and provide continuous coaching in dose management to ensure that the concentration of Tacrolimus in the blood of the patient remains at a suitable level. To aid a patient in timing their regular dosage of Tacrolimus, an acceptable timing range may be indicated. If the patient deviates outside of this range - e.g. by taking a Tacrolimus dose too early or too late - there is a risk that the concentration in the blood of the patient may drop or elevate to an unsafe level.

Summary

An acceptable timing range may be indicated to a patient, e.g. on a patient's device. Although this timing range may be safe in the majority of instances, it is possible that deviations from the optimal timing may result in tacrolimus concentrations falling too low, e.g. if the separation in time of consecutive doses is large enough to create significant deviations, which increases the risk of rejection. It is also possible that deviations from the optimal timing may result in tacrolimus concentrations rising too high, which can be toxic to patients.

According to a first aspect, there is provided a data processing system comprising at least one processor and at least one memory, the at least one processor being configured to execute a set of computer readable instructions to cause the following steps to be performed: providing to a user, an indication of a timing for repeated administration of tacrolimus to a patient; providing to the user, an indication of a range around the timing at which a dose of tacrolimus is to be taken; predicting a concentration of tacrolimus in serum of a patient based on an administered dosage of tacrolimus, a first measured tacrolimus concentration in the serum of the patient, and an indication of a weight of the patient, wherein the predicted concentration corresponds to a time following a measurement time of the first measured tacrolimus concentration; calculating a baseline tacrolimus concentration value using an average of the predicted concentration of tacrolimus in serum and at least one measured tacrolimus concentration in the serum of the patient; calculating a deviation of a further measurement of the tacrolimus concentration in serum of the patient from the baseline tacrolimus concentration value; and in response to determining that the deviation exceeds a predefined limit: reducing the range and providing, to the user, an indication of the reduced range in which a further dose of tacrolimus is to be taken; and transmitting an alert message.

The Tac levels are predicted based upon a previous measurement of the Tac concentration in serum of the patient and based upon the current dosage and body weight of the patient. This predication is then used, along with one or more earlier measurement, to establish a baseline concentration value against which deviations can be measured. If a new measurement indicates a significant deviation from the baseline concentration value, where this deviation is above a threshold, the acceptable range is reduced an alert is transmitted. In this way, greater timing discipline is enforced if large deviations from the baseline are measured.

According to a second aspect, there is provided a computer implemented method comprising: providing to a user, an indication of a timing for repeated administration of tacrolimus to a patient; providing to the user, an indication of a range around the timing at which a dose of tacrolimus is to be taken; predicting a concentration of tacrolimus in serum of a patient based on an administered dosage of tacrolimus, a first measured tacrolimus concentration in the serum of the patient, and an indication of a weight of the patient, wherein the predicted concentration corresponds to a time following a measurement time of the first measured tacrolimus concentration; calculating a baseline tacrolimus concentration value using an average of the predicted concentration of tacrolimus in serum and at least one measured tacrolimus concentration in the serum of the patient; calculating a deviation of a further measurement of the tacrolimus concentration in serum of the patient from the baseline tacrolimus concentration value; and in response to determining that the deviation exceeds a predefined limit: reducing the range and providing, to the user, an indication of the reduced range in which a further dose of tacrolimus is to be taken; and transmitting an alert message.

In some embodiments, the method comprises determining a dosage level of Tacrolimus to be taken by the patient by applying an exponential decay function starting from an initial dosage level of Tacrolimus at a start of treatment to determine the dosage level, wherein the decay rate is dependent upon a weight of the patient.

In some embodiments, the applying the dose predictor to determine the dosage level further comprises applying a Bayesian update function to an output of the exponential decay function based on: a further predicted concentration of tacrolimus in serum; and an earlier output of the Bayesian update function.

In some embodiments, the applying the dose predictor to determine the dosage level further comprises: rounding an output of the Bayesian update function to one of a set of available dosage amounts for tacrolimus.

In some embodiments, the method comprises: applying the dose predictor to determine a dosage level for each of a predefined number of initial doses of tacrolimus.

In some embodiments, the method comprises, in response to determining that the predefined number of initial doses of tacrolimus have been administered, performing the steps of: calculating the baseline tacrolimus concentration value; calculating the deviation of the further measurement; and determining that the deviation exceeds a predefined limit.

In some embodiments, the at least one measured tacrolimus concentration in the serum of the patient comprises a plurality of measured Tacrolimus concentration values.

In some embodiments, the method comprises: providing to the user, an indication of a further range around the timing, the further range being smaller than the range; and in response to determining that the deviation exceeds the predefined amount, reducing the further range and providing the user an indication of the reduced further range.

In some embodiments, the method comprises: in response to receiving user input indicating a dose timing that falls within the range, causing a first message to be displayed on a display of the data processing system.

In some embodiments, the method comprises: in response to receiving user input indicating a second dose timing that falls outside the acceptable range, performing at least one of: transmitting a further alert message; and causing a second message to be displayed on display of the data processing system.

In some embodiments, the method comprises in response to receiving further user input indicating a third dose timing that falls outside of the further range but within the range, causing a third message to be displayed on a display of the data processing system.

In some embodiments, wherein the at least one processor is configured to execute the set of computer readable instructions to check whether the deviation exceeds the predefined limit in response to determining that the baseline tacrolimus concentration value is between an upper bound and a lower bound.

In some embodiments, the method comprises: calculating a deviation in a measured tacrolimus concentration of the at least one measured tacrolimus concentration from an earlier value of the baseline tacrolimus concentration; and performing the steps of reducing the range and transmitting an alert message in response to determining that the deviation from the earlier value exceeds a further predefined limit.

In some embodiments, the method comprises: checking that a deviation in measured concentration from an earlier baseline tacrolimus concentration value did not exceed the predefined limit; and performing the steps of reducing the range and transmitting an alert message in response to determining that the deviation from the earlier baseline tacrolimus concentration value did not exceed the predefined limit.

In some embodiments, the step of predicting the concentration of tacrolimus in serum of a patient is performed in dependence upon an indication of the clearance time for a Tac dose for the patient.

In some embodiments, the step of predicting a concentration of tacrolimus in serum of the patient is performed based on an earlier predicted tacrolimus concentration value.

In some embodiments, the method comprises: for each of a set of a number of doses tacrolimus, determining a deviation in timing of the respective dose from the timing for repeated administration of tacrolimus to the patient; determining for each of the plurality of time periods, an average of the deviations in timing associated with each of a plurality of the doses taken within the respective time period; and setting the range around the timing at which a dose of tacrolimus is to be taken in dependence upon the lowest of the averages for each of the plurality of time periods.

According to a third aspect, there is provided a computer program comprising computer readable instructions, which when executed by at least one processor cause a method according to the second aspect or any embodiment thereof to be performed.

According to fourth aspect, there is provided a non-transitory computer readable medium storing the computer program according to the third aspect.

According to a fifth aspect, there is provided a data processing system comprising at least one processor and at least one memory, the at least one processor being configured to execute a set of computer readable instructions to cause the following steps to be performed: determine an initial estimate for a dosage level of tacrolimus to be taken by a patient during a stage of treatment by applying an exponential decay function starting from an initial dosage level of Tacrolimus at a start of treatment to determine the dosage level of tacrolimus during the stage of treatment; applying a update function to an output of the exponential decay function based on: a predicated concentration of tacrolimus in serum; and an earlier output of the update function; and rounding an output of the update function to one of a set of available dosage amounts for tacrolimus; and providing to a user an indication of the one of a set of available dosage amounts.

According to a sixth aspect, there is provided a computer implemented method comprising: determining an initial estimate for a dosage level of tacrolimus to be taken by a patient during a stage of treatment by applying an exponential decay function starting from an initial dosage level of Tacrolimus at a start of treatment to determine the dosage level of tacrolimus during the stage of treatment; applying an update function to an output of the exponential decay function based on: a predicated concentration of tacrolimus in serum; and an earlier output of the update function; and rounding an output of the update function to one of a set of available dosage amounts for tacrolimus; and providing to a user an indication of the one of a set of available dosage amounts.

According to a seventh aspect, there is provided a computer program comprising computer readable instructions, which when executed by at least one processor cause a method according to the sixth aspect or any embodiment thereof to be performed.

According to an eighth aspect, there is provided a non-transitory computer readable medium storing the computer program according to the seventh aspect.

The fifth to eighth aspects may be combined with any of the features of the first to fourth aspects.

Brief Description of Drawings

Arrangements of the present invention will be understood and appreciated more fully from the following detailed description, made by way of example only and taken in conjunction with drawings in which:

Figure 1 illustrates a computing device according to embodiments;
Figure 2 illustrates a computer system comprising multiple computing device communicating over a network;
Figure 3 illustrates a dosing schedule for providing regular doses to a patient, along with an indication of the optimal timing an acceptable range for taking this doses;
Figure 4 illustrates a number of coaching bands centred on the optimal timing for a dose of tacrolimus;
Figure 5 illustrates an example of the timing of each of a number of doses;
Figure 6 illustrates an example method for managing the timing ranges for the doses of tacrolimus;
Figure 7A illustrates an example of a data table showing the predicted and measured tacrolimus values;
Figure 7B illustrates an example of a data table show the relationship between clearance time and a set of scaler values provided as inputs to the predictor;
Figure 8 illustrates an example of the timing of each of a number of doses, where the ranges are reduced in size at a point during the time range shown;
Figure 9 illustrates an example method for determining the dosage of tacrolimus based on an exponential decay function and a function;
Figure 10 illustrates data showing how parameters of the dose predictor function are related to the weight class of the patient and showing the initial estimate of the ideal Tac dose calculated from the exponential decay function;
Figure 11 illustrates example data tables showing the updated ideal Tac doses determined using the function and the rounded values of the updated ideal Tac doses;
Figure 12 as illustrates the Tac predictor, providing a predicted level of Tac for use by a patient coaching feature and a patient dose predictor;
Figure 13 illustrates an example method for determining a predicted Tac value;
Figure 14 illustrates an example of the receipt of input values associated with each measurement to a system and the processing of these values by different software modules;
Figure 15 illustrates an example of the measured vs predicted concentration of Tac over a series of measurements; and
Figure 16 illustrates an example method for updating the best_time associated with a patient and updating the coaching band width in dependence upon the updated best_time.

Detailed Description

Embodiments of the application relate to a system and method for managing dosage timing of Tacrolimus (hereafter referred to as 'Tac'). The system comprises at least one computer device.

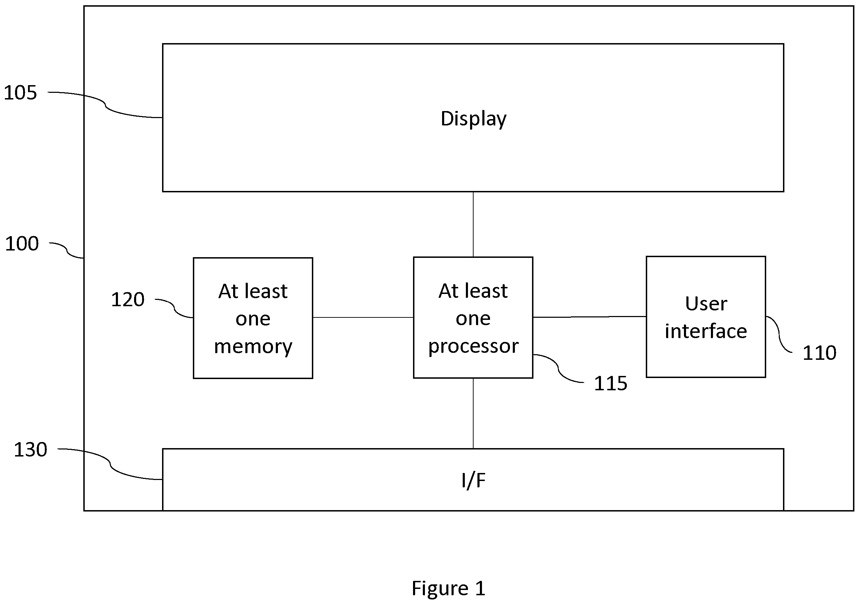

Reference is made to Figure 1, which illustrates a computing device 100 forming part of the data processing system. The computing device 100 may be a mobile user equipment (UE), a personal computer (PC), a terminal or workstation, a server, or some other form of device.

The computing device 100 comprises an interface 130 over which it sends and receives signals. The interface 130 may be a wireless interface. For instance, the interface 130 may comprise a wired interface for connection to a wired network (e.g. a local area network and/or the internet). Alternatively or in addition, the interface 130 may comprise transceiver apparatus configured to send and receive communications over a radio interface. The transceiver apparatus may be provided, for example, by means of a radio part and associated antenna arrangement. The antenna arrangement may be arranged internally or externally to the device 100.

The device 100 is provided with at least one processor 115, and at least one memory 120, which are in communication with one another. The at least one memory 120 comprises a hard drive and random access memory (RAM). The at least one memory 120 stores executable instructions which, when executed by the at least one processor 115, causes the processor 115 to perform the steps described herein as being performed by the device 100. The at least one memory 120 also stores data that may be operated on by the processor 115 and data produced by the processor 115 when performing the operations described herein.

A user controls the operation of the device 100 by means of a suitable user interface 110, such as key pad 110, or by voice commands. A display 105 may be included on the device 100 for displaying visual content to a user. The device 100 may also comprise a speaker for providing audio content.

In some example embodiments, the data processing system for performing the steps described may be provided by the computing device 100. However, such a device 100 may be part of a larger system involved in the management of Tac doses, where that system comprises multiple devices.

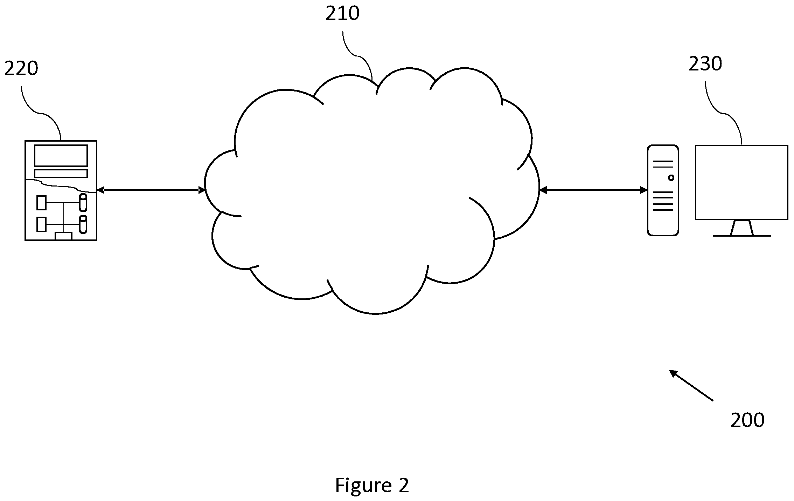

Reference is made to Figure 2, which illustrates an example data processing system 200 according to example embodiments of the application. The data processing system 200 comprises a first computing device 220 and a second computing device 230. Each of the devices 220, 230 may be provided according to the example computing device 100 shown in Figure 1. The first computing device 220 and the second computing device 230 may communicate with one another over a network 210. The first computing device 220 may be a user device 220 belonging to patient. The user may input information into the user device 220 via an app provided on the user device 220. The information input by the user includes an indication of the timing of each dose of Tacrolimus. The information may also include further information about the user, such as the user's weight, age or other characteristics relating to the user. The information may include information relating to the dosage amount of each of the doses of Tac. The second computing device 230 may be a device belonging to a clinic or a testing centre. The second computing device 230 may receive information input by a medical staff member (e.g. a doctor or test centre employee). The information may include blood test results comprising a measurement of Tac concentration in the patient's blood. This information may then be transported over the network 210.

Reference below is made to operations performed by a data processing system. This system may be understood to refer to a single device, such as device 100 or a system comprising a plurality of devices. Reference below to operations performed by such a system or device are understood to refer to operations performed by at least one processor in response to execution of a set of computer readable instructions stored in memory of the device or system.

The patient consumes the Tac dose by ingesting a pill containing the Tac. According to a dosing schedule for administering Tac to a patient, a patient may be required to consume a dose of Tac once at regular intervals. For example, the patient is typically required to consume a dose of Tac every 12 hours. In order to maintain a safe level of Tac, the patient is required to consume the Tac within a safe time window.

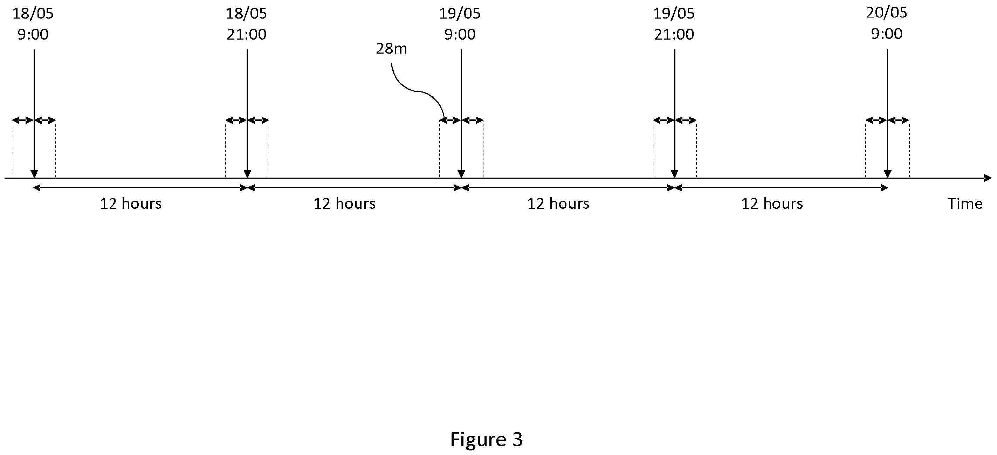

Reference is made to Figure 3, which illustrates a dosing schedule for administering Tac to a patient. Such a dosing schedule is stored in memory 120 of a device 100. The at least one processor 115 controls the display 105 of the device 100 to provide an indication of the dosing schedule, instructing the patient as to when they should consume each dose.

As shown in Figure 3, doses are scheduled to be consumed by a patient at each of a set of regular intervals. In this example, two doses are scheduled for each day, with each dose being scheduled to be 12 hours apart. A first dose shown is scheduled to be taken at 9:00 on 18th May, a second dose is scheduled to be taken at 21:00 on 18th May, and a third dose is scheduled to be taken at 9:00 on 19th May. In Figure 3, the time points are indicated at which each dose should be taken. As shown in Figure 3, a certain window is defined around each time point at which a dose is scheduled. The windows may otherwise be referred to as 'coaching bands', 'ranges' or 'acceptable ranges'.

Each window has upper and lower boundaries set at initial values that are a percentage of a quantity that may be referred to as the 'best_time'. In example embodiments, the upper and lower boundaries may be displaced by 1.4 x best_time from the time point on which the window is centred. The Best_time refers to the lowest of the average deviations recorded over a number of predefined time periods. For example, the system may over a 30 day period, determine for each dose taken within the 30 day period, the deviation in timing of the dose from the respective time point at which that dose was scheduled to be consumed. The system calculates the average deviation for the 30 day period by calculating the average of the timing deviations for each dose. For each 30 day period over the course of the treatment, the system calculates the average deviation. The best_time is set to be equal to the lowest average deviation for each of the 30 day periods that have elapsed so far. If a lower average deviation is recorded for a new 30 day period, the system updates the Best_time to a lower value, and reduces the width of the window.

In the example, shown in Figure 3, each window has upper and low boundaries that are 28 minutes from the time point on which the window is centred (reflecting a best_time of 20 minutes). For example, a first dose is scheduled to be administered on 19th May at 9:00. The lower boundary of the window centred on this time point is at 8:32 am, whilst the upper boundary of the window centred on this time point is at 9:28 am. The patient is required to consume the dose of Tac that is scheduled for consumption at 9:00 am on 19th May within the relevant window, i.e. after 8:32 am and prior to 9:28 am.

When a patient consumes a dose of Tac, the patient inputs into a device 100, an indication of the time at which they consumed the dose. The device 100 determines whether the time falls within or outside the acceptable window defined around the point in time at which the dose is scheduled to be taken. For example, if the dose was scheduled for 9:00 am on 19th May, and the device 100 receives an input indicating that the patient consumed the dose at 9:15 am, the device 100 may determine that the dose was administered at the appropriate time. However, if the device 100 receives an input indicating that the patient consumed the dose at 9:30 am, the device 100 may determine that the dose was not administered at the appropriate time. In this case, the device 100 may respond by advising professional help and immediate attention. The device 100 may respond to the indication that the dose falls outside the window by transmit an alert message, which may be received by a medical staff member, e.g. a physician.

In some embodiments, multiple windows may be defined around a time point. A first window may be the type of window discussed above with respect to Figure 3, and correspond to an acceptable time range for consumption of a Tac dose. The second window may correspond to a recommended time range for consumption of a Tac dose. For both windows, the boundaries may be set based upon the best_time associated with the window. As discussed above, the boundaries of the first window may be set at 1.4 x best_time. The boundaries of the second window may be set at 1.25 x best_time.

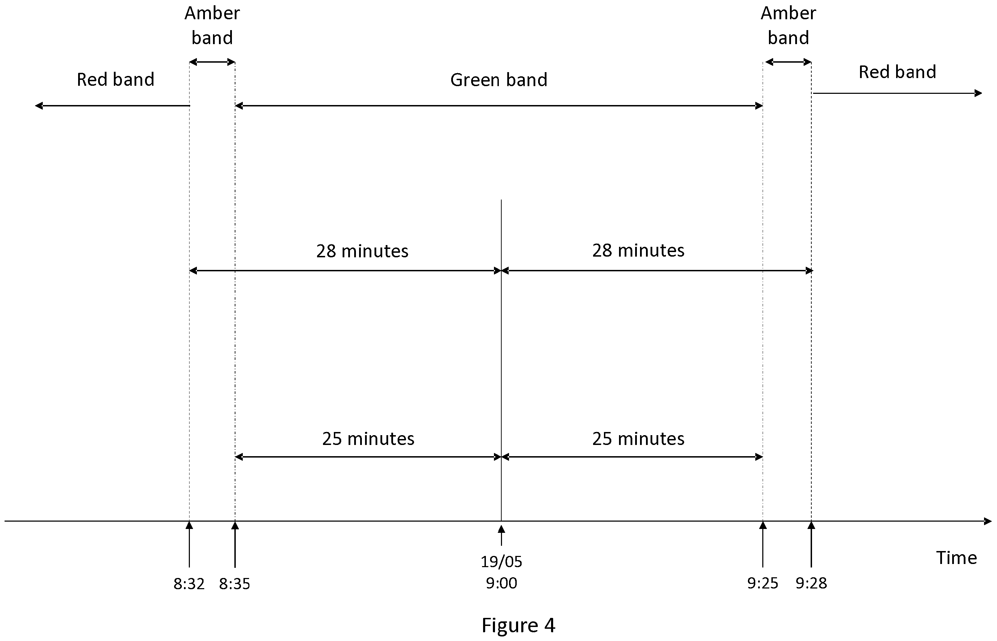

Reference is made to Figure 4, which illustrates a first and second window, which are both centred upon a time point for administration of a dose of Tac. As shown in Figure 4, the time point is at 19th May at 9:00. As in Figure 3, a first time window is shown with boundaries that ± 28 minutes from the time point. Additionally, a second time window is shown with boundaries that are ± 25 minutes from the time point. The second time window defines a first coaching band, which is referred to herein as the green coaching band or green band. In response to receipt of an indication that the dose was consumed by the patient at a time (e.g. 9:15am) falling within the green band, the processor 115 may control the display 105 to display a first message. The first message may comprise positive behavioural reinforcement for a user. The first and second time windows define a second coaching band, which is referred to herein as the amber coaching band or amber band. The amber band falls within the first time window, but outside of the second time window. In response to receipt of an indication that the dose was consumed by the patient at a time falling within the amber band (e.g. at 9:26 am), the processor 115 may control the display 105 to display a second message that is different from the first message. The second message may comprise negative, or at least less positive, reinforcement for a user. The first time window defines a third coaching band, which is referred to herein as the red coaching band or red band. The red band falls outside the first time window. In response to receipt of an indication that the dose was consumed by the patient at a time falling within the red band (e.g. at 9:30am), the processor 115 may control the display 105 to display a third message that is different from the second message and first message. The device 100 may also respond to the indication that the dose falls within the red band by transmitting an alert message, e.g. to a physician.

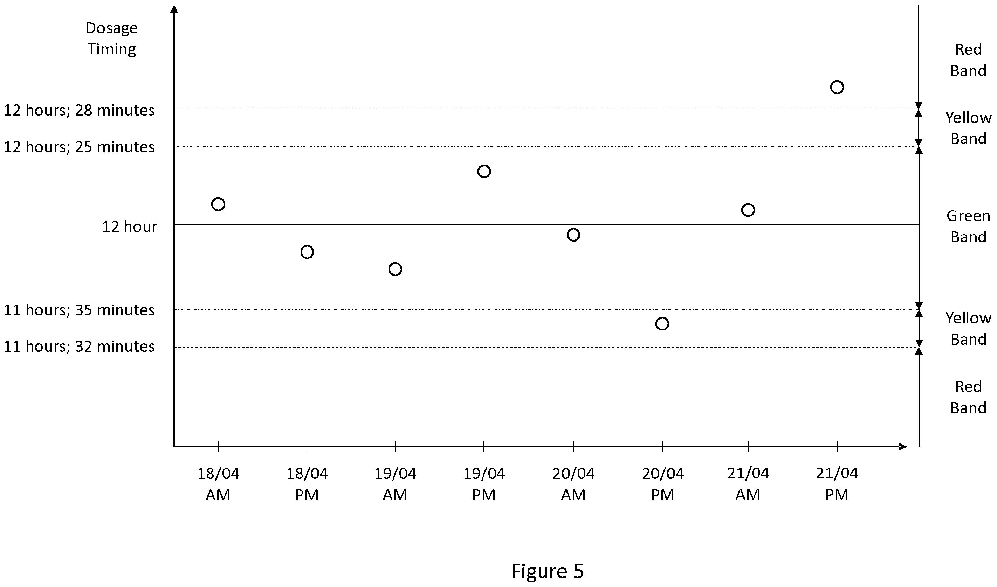

Reference is made to Figure 5, which illustrates an example of the timing of different doses of a tacrolimus consumed by a patient. The Figure indicates how much the timing of each dose deviates from the time points in the dose schedule, and indicates the boundaries of the time windows defining each of the bands. As shown in Figure 5, six of the doses (taken from 18/04 am to 20/04 am and on 21/04 am) are each taken within the green band. One of the doses (taken on 20/04 pm) is taken within the amber band, and one of the doses (taken on 21/04 pm) is taken within the red band. The information displayed in Figure 5 may be shown on the display 105 of the device 100.

According to embodiments, the device 100 may determine to reduce the ranges represented by the time window/s discussed above if it is determined that these ranges are insufficiently narrow to ensure that safe Tac concentrations are maintained. This determination is carried out on the basis of measurements of the Tac concentration in the blood of a patient. The measurement of the Tac concentration in the blood of the patient are also referred to herein as measurements of the concentration in serum.

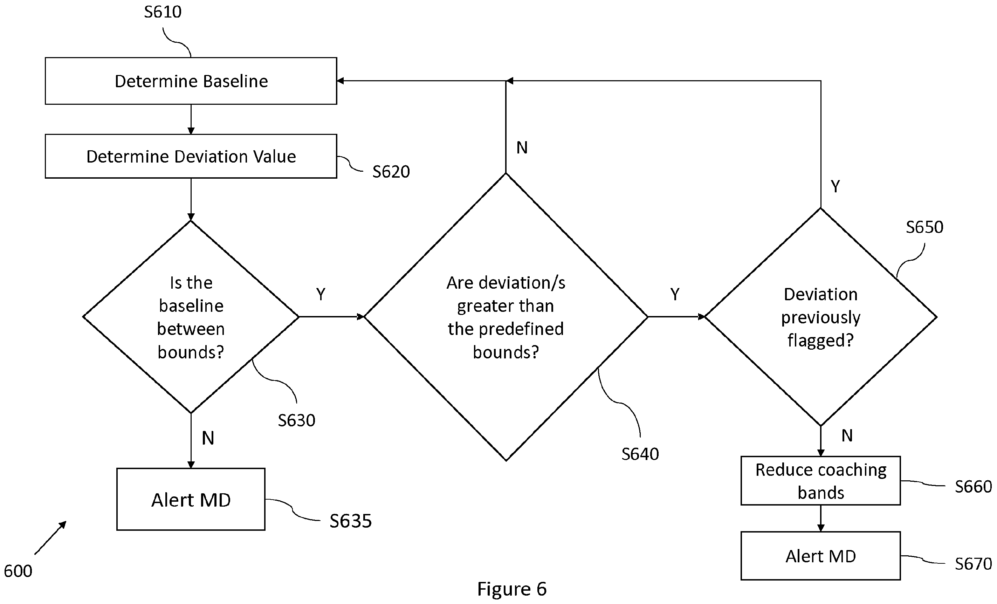

Reference is made to Figure 6, which illustrates a method 600 for determining when to reduce the timing ranges for administering a dose to a patient. The method 600 is implemented in a data processing system, e.g. system 100 or system 200. The method 600 comprises at S610, determining a baseline value of Tac concentration in the blood of the patient. The baseline value represents an average Tac concentration based on at least one previous blood sample and based on a predicted Tac concentration determined by the data processing system based on a prediction algorithm. This provides an effective linear filter that smooths out movements in the Tac concentration, whilst providing a baseline value based on the most recent predictions and measurements of the Tac concentrations available. In example embodiments, the baseline value is given by:

$$Baseline\ value = \frac{(Tac\_prediction\ +\ Latest\_Tac\_Value\ +\ Previous\_Tac\_Value)}{3} \qquad \text{Equation 1}$$

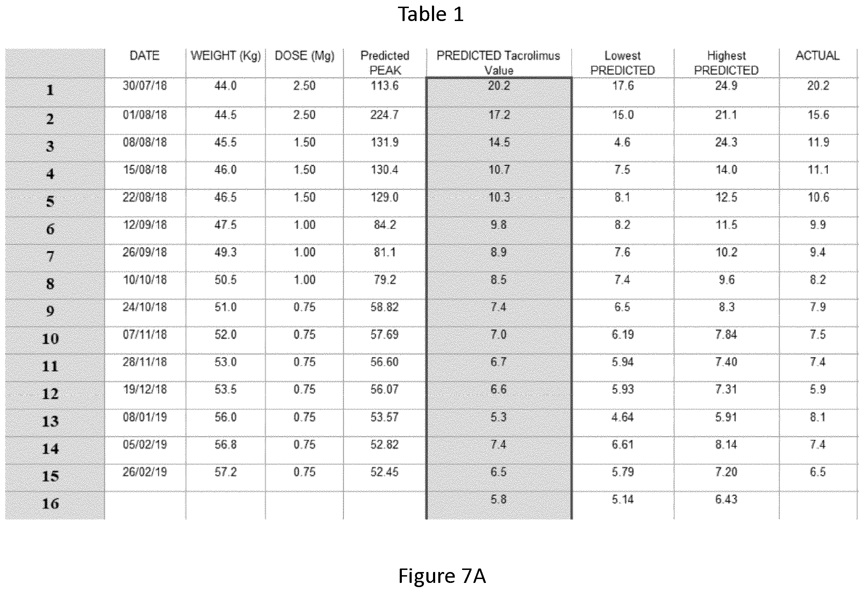

In Equation 1, the "Latest _Tac_Value" and the "Previous_Tac_Value" represent the concentration of Tac determined from the two most recent blood samples. Reference is made to Figure 7A, which illustrates an example of series of measured Tac concentrations (shown in the 'Actual' column), along with the dose and weight for a patient. If, for example, a baseline value is to be determined for the 16th measurement, the Latest_Tac_Value for this measurement is 6.5 mg/L (which is the concentration that is measured on the 26/02/19) and the Previous_Tac_Value is 7.4 mg/L (which is the concentration that is measured on 05/02/19). The data processing system receives the indications of the Latest_Tac_Value and the Previous_Tac_Value on the basis of blood measurement results (which may be provided by user input or directly based on test measurement).

In Equation 1, the parameter "Tac_prediction" represents a predicted concentration of Tac. This predicted concentration may otherwise be referred to as the predicted Tac value. The predicted Tac values are given Table 1, along with the 95% confidence intervals. Each predicated Tac value is given by:

$$Tac prediction = PP * ATV * Scaler / (PP + Previous Tac Level Prediction) \qquad \text{Equation 2}$$

Here PP is the predicted peak, which is the peak predicted Tac level in the bloodstream. The predicted peak is proportional to the dose consumed by a patient and inversely proportional to the weight of the patient. The predicted peak is given by:

$$Predicted Peak = (Dose * 200) / (Weight * 5\%) \qquad \text{Equation 3}$$

In Equation 3, the dose is the most recently consumed dose amount of Tac, and the weight is the most recently measured weight of the patient. Table 1 indicates how the dosage level of Tac consumed by the patient and the weight of the patient change over the course of the patient's treatment.

The predicted peak value that is used as an input into Equation 2 is based upon the most recently measured weight of the patient. As shown in table 1, the predicted peak of the patient determined based on data available prior to the 16th measurement is equal to = (0.75*200)/(57.2*0.05) = 52.45. The predicted peak value used in Equation 2 to predict the Tac concentration is that calculated by the data processing system based on the most recent weight measurement and dosage prior to the date of the 16th measurement (i.e. the weight and dosage level on 26/02/19). Therefore, the predicted peak value that is used in Equation 2 to predict the Tac concentration level is equal to = (0.75*200)/(57.2*0.05) = 52.45.

The 'ATV' in Equation 2 refers to the Actual Tac Value, which is the most recently measured Tac concentration value. Supposing that, for the patient whose data is shown in table 1 in Figure 7A, it is desired to predict the concentration of Tac on 26/02/19, the most recent Tac value is the value measured on 26/02/19, which is given by 6.5 mg/L. Therefore, 6.5 mg/L is the ATV for the prediction of the concentration to be made following 26/02/19.

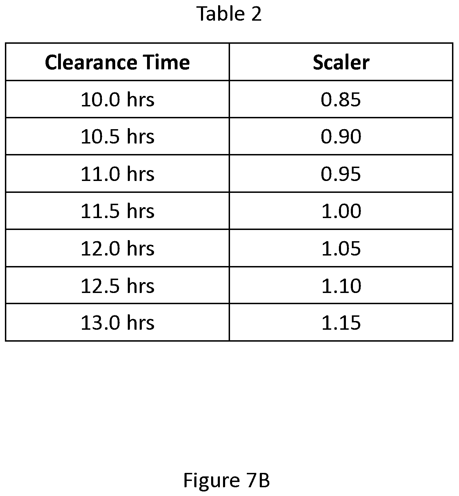

The 'Scaler' in Equation 2 is a value that is dependent upon the clearance time for the patient. The clearance time is the taken for a Tac dose to clear the body. The clearance Time is determined based upon indications of the concentration of Tac levels in the bloodstream. A Kalman filter or another filter with similar effect may be used by the data processing system to determine this. Once the clearance time is known, this is rounded to nearest 0.5 hour and converted into a scalar value as indicated in table 2 shown in Figure 7B. The scalar values shown in table 2 have been determined empirically by identifying the linear relationship between clearance time and the measured Tac level. For 66% of all adults, the clearance time is approx. 11.5 hours, which translates into a scaler value of 1.00.

The 'PreviousTacLevelPrediction' in Equation 2 is the most recent value of the quantity 'Tacprediction' that was determined for the patient by the data processing system. For example, if the predicted Tac value was determined from Equation 2 as being equal to 6.5 mg/L on 26/02/19, when determining the predicted Tac value following 26/02/19, 6.5 mg/L is taken to be value for 'PreviousTacLevelPrediction'.

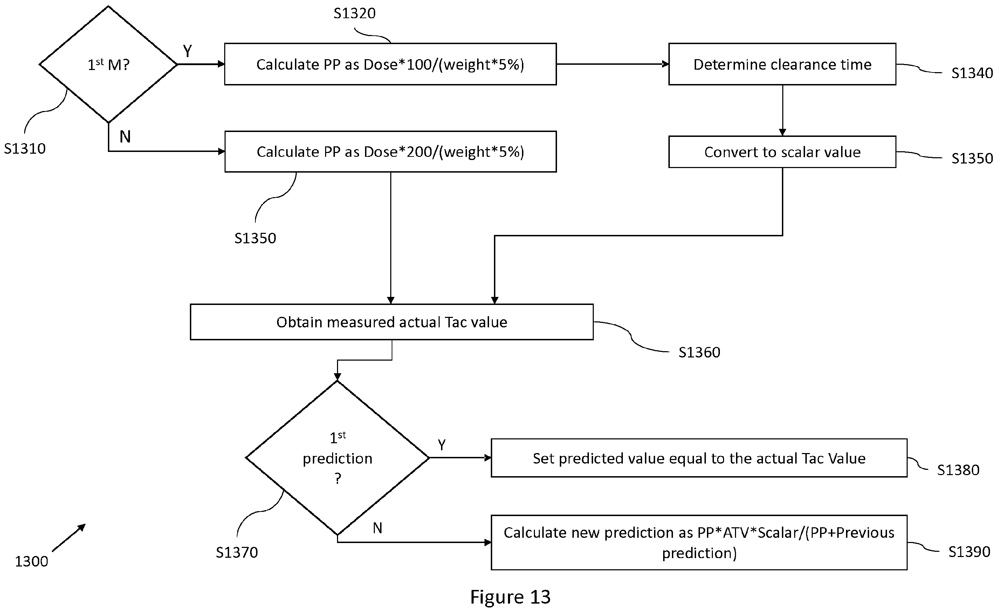

Reference is made to Figure 13, which illustrates a method 1300 performed by the data processing system to predict the concentration of Tac. The system determines at S1310 whether the measurement collected by the data processing system is the first of the collected measurements (at which a patient's weight and Tac levels are measured) during the course of administration of Tac. If the measurement is the first measurement, at S1320, the system calculates the predicted peak as being equal to half the value represented by Equation 2. If the measurement is not the first measurement, at S1330, the system calculates the predicted peak as being equal to the value represented by Equation 2. At S1340, the system determines the clearance time for the patient. At S1350, at the system converts the clearance time to a scalar value. At S1360, the actual Tac level is determined based on a blood sample collected from the patient. At S1370, the system determines whether or not the prediction being generated by method 1300 is the first of the prediction of the TAC level generated by the patient or whether a prediction has already been made. If the prediction is the first of the predictions, the method 1300 proceeds to S1380, at which point the predicted value is set equal to the actual Tac value that was obtained at S1360. If the prediction is not the first of the predictions, at S1390, the system calculates the new prediction by applying Equation 2 to the predicted peak determined at S1320 or S1350, the scalar value for the patient, the Tac value measured at S1360, and the previous prediction of the Tac value. The system stores the predicted Tac value (determined at either S1380 or S1390) for use in producing a further predicted Tac value when a further iteration of the method 1300 is performed.

The data processing system in performing S610, calculates the predicted concentration of Tac by applying Equation 2 to the quantities discussed. Having determined the predicted concentration of Tac, the data processing system then calculates the baseline value by taking an average of the predicted Tac concentration, the Latest_Tac_Value and the Previous_Tac_Value.

Taking the example of predicting the Tac concentration following 26/02/19, the latest Tac value and the previous Tac value, as shown in Table 1, are given by 6.5 mg/L and 7.4 mg/L respectfully. The most recent predicted peak is determined by the data processing system to be equal to 0.75*200/(52.82*0.05) = 52.45. Using this value for the predicted peak, the data processing system then calculates the predicted Tac concentration as being equal to 52.82*6.5*1/(52.82 + 6.5) = 5.8 mg/L. Based on Equation 1, the data processing system, therefore determines the baseline Tac concentration value to be equal to = (5.8 + 6.5+ 7.4)/3 = 6.57 mg/L.

At S620, the data processing system receives a new value for the Tac concentration, which is received via user input. A further quantity representing the deviation of the new Tac concentration from the baseline value is determined by the data processing system. The deviation value is given by

$$Deviation\ value = ABS(New\ Tac\ Value - Baseline)/Baseline \qquad \text{Equation 4}$$

The deviation value, therefore, represents an indication of the change from the baseline Tac concentration. As an example, following 26/02/219, a blood sample may be taken from which it is determined that the Tac concentration is 6.1 mg/L. This value is provided as an input into the data processing system. Additionally, as discussed above, the data processing system determines the baseline value as being equal to 6.57 mg/L. From Equation 4, the data processing system determines the deviation value as being equal = ABS(6.1-6.57)/6.57 = 7.15 %.

After the data processing system has determined the baseline value and the deviation of the newly measured Tac concentration from the baseline, the method 600 proceeds to S630, at which point the data processing system determines whether or not the baseline is within certain predefined bounds. The predefined bounds comprise a lower bound and an upper bound. In embodiments, the lower bound is equal to 5.9 mg/L, whereas the upper bound is given by 9.1 mg/L. These bounds represent safe boundaries for the level of Tac. If the system determines that the baseline level is above the upper bound, this indicates that the levels of Tac may be too high and be toxic to the patient. If the system determines that the baseline level is below the lower bound, this indicates that the levels of Tac may be too low such that the patient is at risk of organ rejection. If the baseline value is determined from Equation 1 to be between the upper and lower bounds, the method 600 proceeds to S640. If the data processing system determines that the baseline Tac concentration falls outside of these bounds, at S635, the system may transmit an alert message (e.g. to alert the physician).

At S640, the data processing system determines whether the calculated deviation of the new Tac concentration from the baseline is greater than a predefined boundary. In embodiments, the predefined boundary is 10% of the baseline value. In response to determining that the deviation exceeds the predefined boundary, the method 600 proceeds to S650.

In embodiments, S640 may also comprise determining whether or not a previously calculated deviation is greater than a further boundary. The previously calculated deviation is in relation to the preceding measurement prior to the measurement from which the new Tac concentration was determined. If a new measurement was carried out on a particular date, the data processing system determines the deviation from the baseline calculated on that date and determines whether or not this deviation is greater than a predefined boundary (e.g. 10%) that applies to the deviation (which may be referred to as the current deviation) in relation to the most recent measurement. In response to determining that the current deviation from the baseline is greater than the predefined boundary, the data processing system examines the deviation associated with the preceding measurement (which may be referred to as the previous deviation) from a baseline established at the preceding measurement. The data processing system determines whether or not the previous deviation is greater than the further predefined boundary (e.g. 4.7%). In response to determining that both the current deviation and the previous deviation exceed their respective predefined boundaries, the method 600 may proceed to S650. Taking account of the previous deviation, in addition to the current deviation, may allow for detection of a large and persistent deviation in the Tac concentrations level.

At S650, the data processing system may check whether or not, in a previous iteration of the method 600 in which a deviation value was calculated at S620, whether or not the conditions in S630 and S640 were met. If so, the action defined in S660 has already been performed, and is not performed again. However, if the data processing system determines at S650 that the conditions defined in S630 and S640 have not yet been met in relation to preceding measurements, the method 600 proceeds to S660.

At S660, the data processing system reduces the coaching bands discussed previously to narrower ranges. This step comprises the processor 115 of the device 100 controlling the display 105 to indicate to the user, the updated conditions regarding dose time.

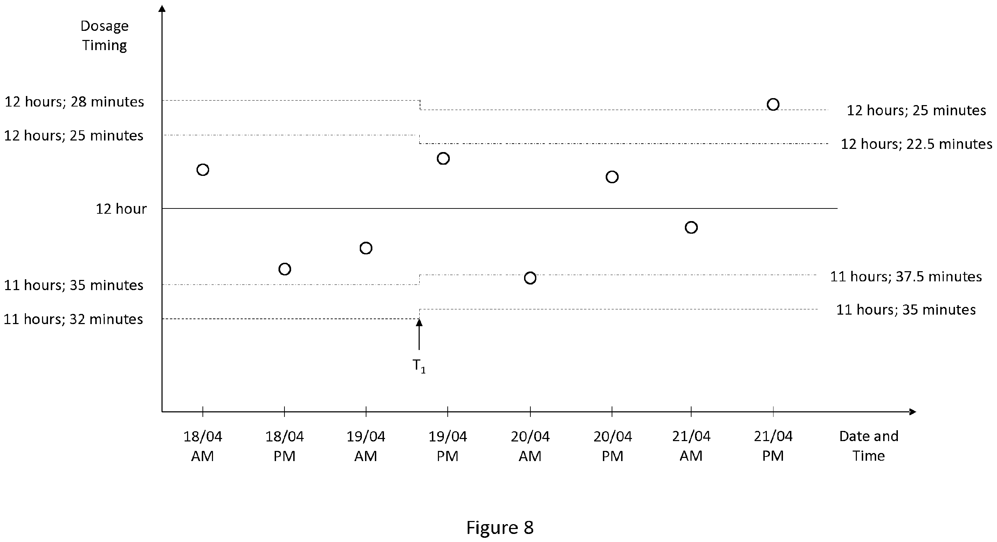

Reference is made to Figure 8, which illustrates how the coaching bands may be reduced in response to determining that a deviation in the Tac concentration exceeded a predefined bound. As shown in Figure 8, the windows have their initial default boundaries, with the upper and lower boundaries of the first window being displaced by 28 minutes from the scheduled time point, and the upper and lower boundaries of the second window being displaced by 25 minutes from the scheduled time point. A number of doses of Tac are shown as being administered to the patient (e.g. at 18/04 am, 18/04 pm, and 19/04) prior to a change in the window ranges. The user records the timing of each of these doses in the device 100, with the processor 115 of the device 100 controlling the display 105 to show the timing of each of these doses in a graphical format. As shown, each of the doses is administered within the boundaries represented by second window, i.e. within the green band.

At a time $T_1$, the data processing system performs the method 600 on the basis of new measurement of Tac concentration, the Tac concentration being received as an input to the data processing system. At time $T_1$, the data processing system determines that the condition at S640 is met and proceeds to S660 to reduce the range represented by each of the windows. The range of each window is reduced by approximately 10%, such that the upper and lower boundaries of the first window are now displaced by 25 minutes from the time point at which a dose of Tac is scheduled for being administered, and the upper and lower boundaries of the second window are displaced by 22.5 minutes from the time point at which a dose of Tac is scheduled for being administered.

The timing of a number of further doses is shown in Figure 8 following the time point $T_1$. One of the doses - which is shown in this example as being administered on 20/04 am - is administered at a time that is within the previously defined boundaries for the second window. However, the time falls outside the second window as defined by the new boundaries, i.e. it falls within the amber band. In response to determining that the time falls outside the boundaries, the device 100 performs the actions defined for the amber band, e.g. the processor 115 controls the display 105 to cause the message associated with the amber band to be displayed.

Another of the doses - which is shown in this example as being administered on 21/04 pm - is administered at a time that is within the previously defined boundaries for the first window. However, the time falls outside the first window as defined by the new boundaries, i.e. it falls within the newly defined red band. In response to determining that the time falls outside the boundaries, the device 100 performs the actions defined for the red band, e.g. the processor 115 controls the display 105 to cause the message associated with the amber range to be displayed. The device 100 may respond to the indication that the dose falls within the red band by transmitting an alert message, e.g. to a physician.

Referring back to Figure 6, in addition to performing the S660, in response to determining at S640 that the deviation/s exceed predefined bounds, S670 is also performed by the system. In this step, the device 200 transmits an alert over a network 210 to the device 230. The alert provides an indication to the physician that the deviation has occurred.

The method 600 described above with reference to Figure 6 may be part of a patient coaching regulator. The patient coaching regulator is used for careful monitoring of the timing of doses to ensure that the Tac concentration in serum of the patient remains at a safe level. The patient method 600, which is based on measuring the deviation from baseline Tac concentration, may be used at a later stage in the Tac dosing schedule when the dosages consumed are of a low level, and the control of the timing, rather than the dose, assumes greater importance in control of Tac levels. Prior to the use of the patient coaching regulator, a dosing predictor may be used to determine and control the dose of Tac to be taken by the patient.

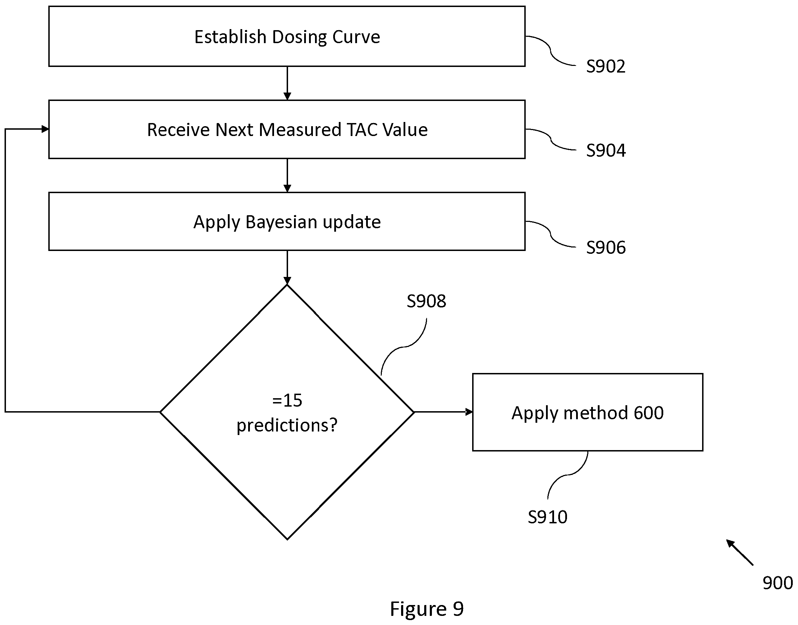

Reference is made to Figure 9, which illustrates a method 900 for determining the dose to be taken by the patient, which is implemented in the data processing, e.g. system 100 or system 200.

At S910, an initial dosing curve is established for the patient. The dosing curve is a negative exponential curve providing an initial estimate of the ideal Tac dose for the patient over time. This initial estimate is provided based upon the patient's weight and an initial concentration of Tac measured in the serum of the patient. The dosing curve is calculated by the system as:

$$Ideal\ Tac\ dose = Initial\ dose * \left((IV - Offset)e^{-DR*M} + Offset\right)/IV \qquad \text{Equation 5}$$

In Equation 5, the IV is the initial value, which is first measured Tac concentration value. For the example data shown in Figure 7A, the Tac concentration value is equal to 20.2 mg/L.

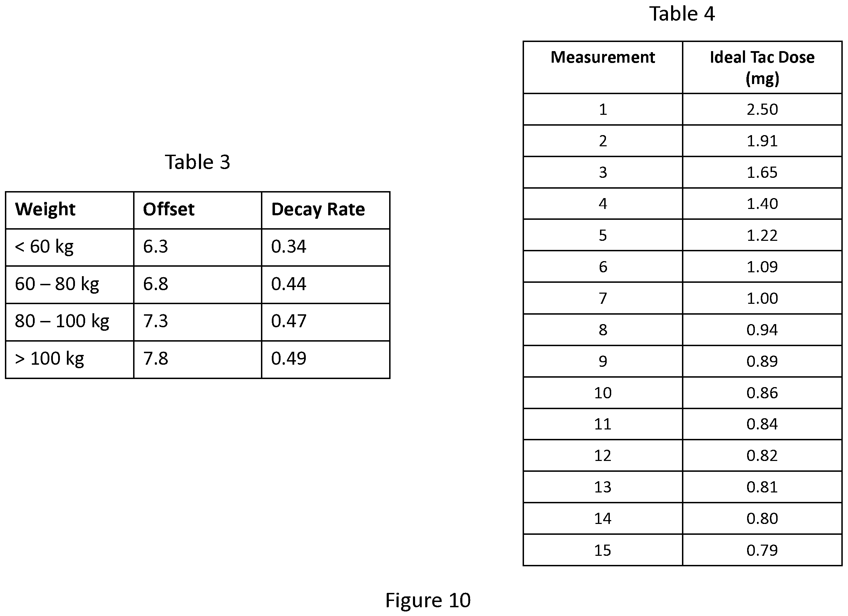

The offset value in Equation 5 is dependent upon the patient's weight. Reference is made to Figure 10, which illustrates a table (Table 3) showing a set of weight classes for the patient and corresponding offset values for the patient. For the example, patient for which a dataset is shown in Figure 7A, the offset value is 6.3, since the patient's weight is less than 60 kg.

In Equation 5, DR provides a decay rate for the negative exponential function. The decay rate is also dependent upon the patient's weight class. As shown in Table 3 of Figure 10, the decay rate for the patient having the dataset shown in Figure 7A is 0.34.

In Equation 5, M refers to the measurement number, which identifies the measurement of Tac made, where the measurements are numbered in chronological order. As shown in Figure 7A, the first 15 Tac measurements are numbered from 1 to 15.

In Equation 5, the initial dose is the first dosage level that is administered to the patient. This dosage level is input into the data processing system and is, for example, a dosage amount that is determined by a physician. In the example shown in table 1 and the example shown in tables 4 to 6, the initial dosage amount is 2.50 mg.

To improve the estimate for an ideal Tac dose for a patient, the data processing system applies an update process, which makes use of future measured Tac values to correct the values derived from dosage curve represented by Equation 5. The update process may be referred to as a Bayesian update process. The Bayesian update process may be referred to as Bayesian predictor or Bayesian function.

At S904, the data processing system receives a measured Tac concentration value, which is input into the data processing system. This measured concentration value is a measurement collected after the first of the collected measurements.

At S906, the data processing system applies an update process to obtain an updated Tac dose based on the dosage curve established at S902 and based on the new measured concentration value obtained at S904. The updated TAC dose (UTD) is given by:

$$UTD = \frac{PTV.ITD}{PTV + PUTD} \qquad \text{Equation 6}$$

PTV is the predicted Tac value which, in accordance with Equation 2, is obtained based on an administered dosage of tacrolimus and an earlier measured tacrolimus concentration in the serum of the patient. ITD is the ideal Tac dose determined from equation 5. PUTD is referred to as the prior updated TAC dose and represents the dosage level at the previous measurement point that was determined from equation 6. If there is no prior updated value determined from equation 6, e.g. because the measurement point is the first point (i.e. is the second measurement point overall) at which equation 6 is applied, PUTD is taken to be equal to the initial starting dose (e.g. 2.50).

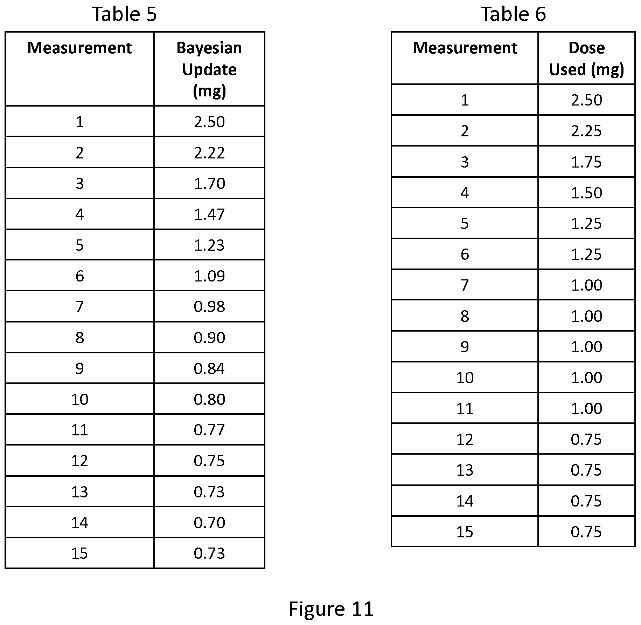

Reference is made to Figure 11, which illustrates two further tables. Table 5 shows a series of values obtained by applying a Bayesian update process based on a series of predicted Tac concentration levels and based on a dosage curve.

The actual dosages administered to the patient depend upon the Tac dose weights available: 0.5, 0.75, 1.0, 2.0, 3.0 mg. Given the available dosages, the dosage administered to the patient must be a multiple of 0.25 mg. Therefore, values shown in table 5 are rounded by the data processing system up to the nearest 0.25 mg, to obtain the values shown in table 6. Such values may be shown on the display 105 of the device 100 to provide indicate to the patient, the dosage to be consumed at each point in time. These dosages are administered to the patient during the time period following each of the measurement. For example, between blood sample measurement 1 and blood sample measurement 2, the patient takes a dose of 2.5 mg of Tac every interval (e.g. 12 hours) defined in the dosing schedule. Between blood sample measurement 9 and 10, the patient takes a dose of 0.75 mg of Tac every interval (e.g. 12 hours defined in the dosage schedule).

Referring back to Figure 9, after having applied the update at S906, the method 900 proceeds to S908, where it is determined whether a predetermined number of doses have been predicted by applying the dosing curve and update process at S906. In the example shown, the number is 15. If not, the method 900 continues by receiving further measurements of TAC concentration and using these to apply an update process to determine further doses. If the predetermined number of doses has been determined, the method 900 proceeds to S910, where the method 600 is applied for controlling the timing at which doses are administered. When the method 600 is applied, the Tac dose may be held constant (e.g. at 0.5 mg).

Discussed above is the method (for which the steps are illustrated in Figure 9) in which the dosing for a patient is determined. This may be referred to as the patient dose predictor. Also discussed above is the method (for which the steps are illustrated in Figure 6) in which the coaching bands are presented to a user and adjusted based on measured and predicted Tac levels. This may be referred to as the patient coaching feature. Both of these methods may be performed together for a single patient in response to the execution of computer readable instructions that form part of a computer program. The method both receive an output of a Tac level predictor.

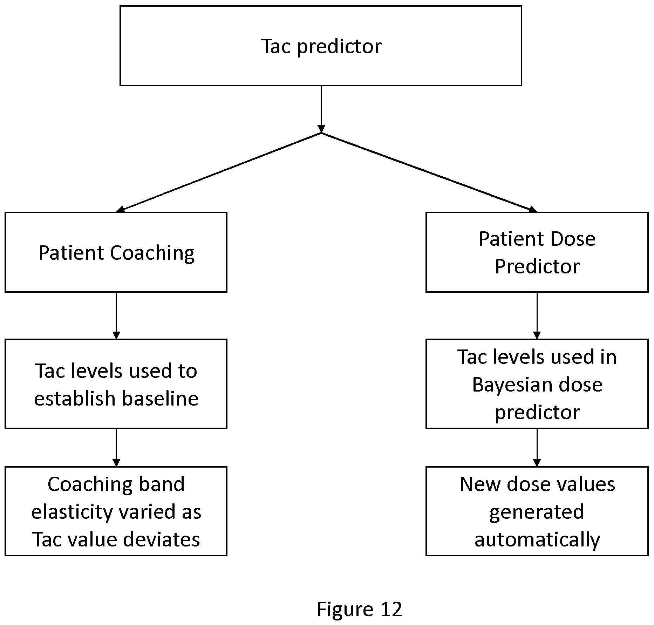

Reference is made to Figure 12, which illustrates how a Tac predictor may be used to provide a predicted Tac level to both a patient coaching feature and an output to a patient dose predictor. The patient coaching feature uses the predicted Tac level to establish a baseline value, which is an average of the predicted Tac level from the patient dose predictor and earlier measured Tac concentration value/s. The patient coaching feature measures the deviation of further measured Tac values from the baseline in order to determine whether the range of the coaching bands should be varied. The patient dose predictor uses the predicted Tac level as part of the dose predictor, so as to generate new dose values automatically.

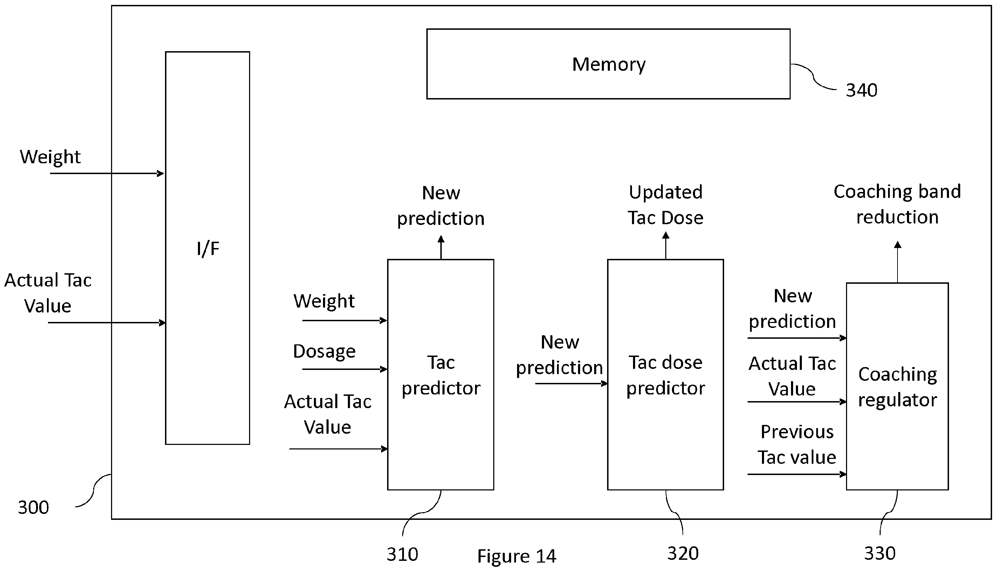

Reference is made to Figure 14, which illustrates an example of a system 300 according to embodiments and shows the parameters received at the system at each measurement point (e.g. each of measurements 1 to 15 shown in table 1. The system 300 may be a system 100 or system 200. A number of software modules run on at least one processor of the system 300. These software modules include the Tac predictor 310, a Tac dose predictor 320, and a coaching regulator 330. The system 300 includes a memory 340, which stores parameters, e.g. scaler value, initial measured Tac value, which remain constant over multiple measurements.

As shown, at each measurement point, an indication of the weight of the patient, and an indication of the measured Tac concentration for the patient are received at the system 300. These values may be received via user input. These values are used, along with an indication of the most recent dosage consumed by the patient, are used by the Tac predictor 310 to predict the Tac concentration at an upcoming measurement point. A new weight indication and measured Tac concentration are received and processed by the Tac predictor for each iteration of method 1300 to produce a new Tac level prediction. The Tac dose predictor 320 may use the predictions when performing the steps S904 and S906 of method 900 to determine a Tac dose to be administered to a patient. The coaching regulator 330 may, when performing each iteration of method 600, use one of the predictions, along with the measured Tac values to reduce the coaching bands.

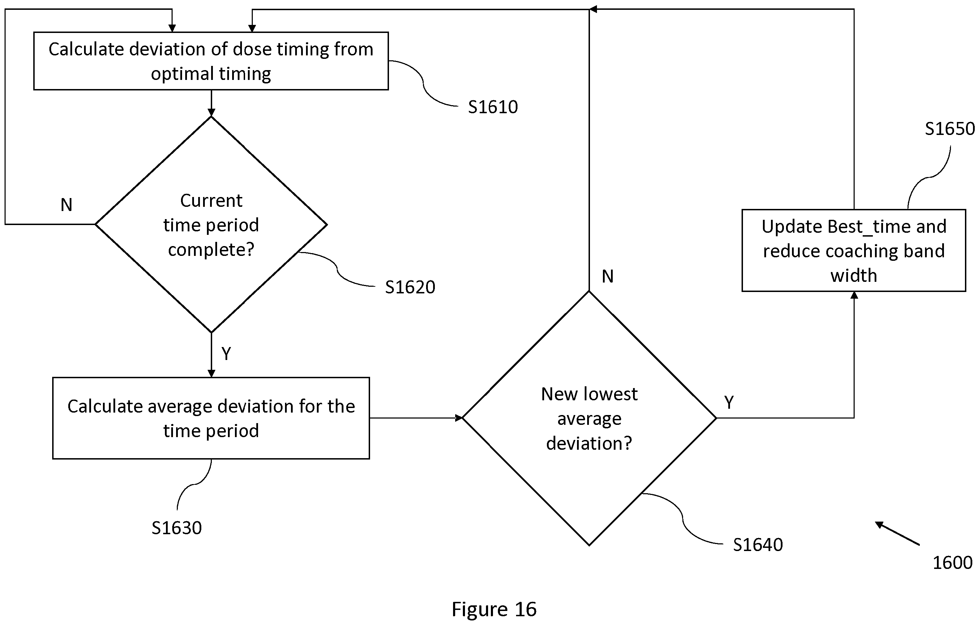

Reference is made to Figure 16, which illustrates an example method 1600 performed by the system for using the deviation of the dose from the optimal timing to determine the width of the coaching band/s. The method 1600 may be performed by the system in parallel with the method 600 to adjust the width of the coaching bands.

At S1610, the system receives (e.g. from user input) an indication of the time at which a dose was taken by the patient. The system calculates the deviation of this time from the scheduled time for that dose and stores this deviation in memory.

At S1620, the system determines whether a time period during which a plurality of doses (including the most recent dose) are scheduled to be consumed has expired. The time period may, for example, comprise 30 days in which doses are scheduled, in accordance with a schedule stored the system, to be taken by the patient every 12 hours. If the time period is not complete, the method 1600 proceeds again to S1610, at which point the average deviation is determined for the next dose. If the time period is complete, the method 1600 proceeds to S1630.

At S1630, the system calculate the average deviation for the time period (which was determined to be complete at S1620) based on the deviation values calculated at S1610.

At S1640, the system compares the average deviation determined at S1630 to the average deviation determined for earlier time periods preceding the most recent time period. If the average deviation determined for the most recent time period is lower than the average deviations for any earlier time period, at S1650 the system updates the best_time to be set equal to average deviation calculated at S1630. The system reduces the coaching band width to correspond to the updated best_time, and provides, to the user, an indication of the reduced range in which the dose of tacrolimus is to be taken.

If the average deviation determined at S1630 is not the lowest average deviation for the time periods, method 1600 proceeds to perform steps S1610, S1620, and S1630 in relation to a further time period.

Table 1 of Figure 7A illustrates an example set of predicted Tac concentration values using the Tac predictor (i.e. by applying equation 2) discussed above applied to a patient having the weight and shown in Table 1 and being provided with the Tac dosage amounts shown in table 1. The predicted Tac concentration values and measured Tac concentration values (shown in the column 'Actual') are provided in mg/L. For this patient, the clearance time is 11.5 hours, which translates to a scaler value of one in Equation 2. Also shown in table 1 are a set of dose values determined for the patient by applying the dosage curve and Bayesian predictor in method 900 to determine the ideal doses, when starting from an initial dose of 2.50 mg.

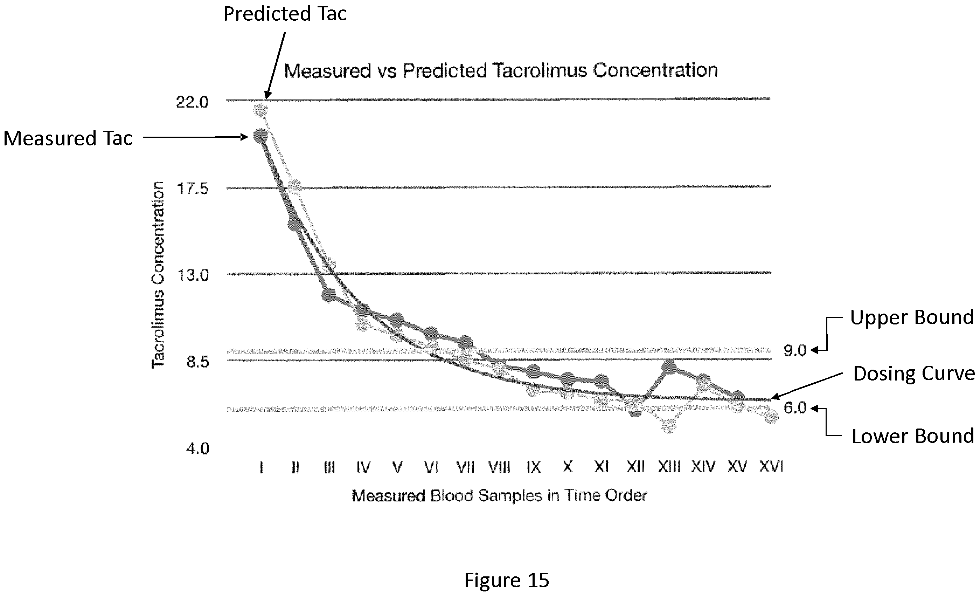

Reference is made to Figure 15, which illustrates an example of a series of predicted Tac values and a series of measured Tac values for a patient. Each predicted or measured Tac value is associated with a blood sample that occurs at a measurement point. The upper and lower bounds taking the values of 9.0 and 6.0 in this example) applied to the baseline Tac value in S630 are also shown in Figure 5. A dosing curve for the patient, which is derived by the system based on Equation 5 is also shown.

Implementations of the subject matter and the operations described in this specification can be realised in digital electronic circuitry, or in computer software, firmware, or hardware, including the structures disclosed in this specification and their structural equivalents, or in combinations of one or more of them. For instance, hardware may include processors, microprocessors, electronic circuitry, electronic components, integrated circuits, etc. Implementations of the subject matter described in this specification can be realised using one or more computer programs, i.e., one or more modules of computer program instructions, encoded on computer storage medium for execution by, or to control the operation of, data processing apparatus. Alternatively or in addition, the program instructions can be encoded on an artificially generated propagated signal, e.g., a machine-generated electrical, optical, or electromagnetic signal that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

While certain arrangements have been described, the arrangements have been presented by way of example only, and are not intended to limit the scope of protection. The inventive concepts described herein may be implemented in a variety of other forms. In addition, various omissions, substitutions and changes to the specific implementations described herein may be made without departing from the scope of protection defined in the following claims.

## Claims

1. A data processing system comprising at least one processor and at least one memory, the at least one processor being configured to execute a set of computer readable instructions to cause the following steps to be performed:

   providing to a user, an indication of a timing for repeated administration of tacrolimus to a patient;
   providing to the user, an indication of a range around the timing at which a dose of tacrolimus is to be taken;
   predicting a concentration of tacrolimus in serum of a patient based on an administered dosage of tacrolimus, a first measured tacrolimus concentration in the serum of the patient, and an indication of a weight of the patient, wherein the predicted concentration corresponds to a time following a measurement time of the first measured tacrolimus concentration;
   calculating a baseline tacrolimus concentration value using an average of the predicted concentration of tacrolimus in serum and at least one measured tacrolimus concentration in the serum of the patient;
   calculating a deviation of a further measurement of the tacrolimus concentration in serum of the patient from the baseline tacrolimus concentration value; and
   in response to determining that the deviation exceeds a predefined limit:

      reducing the range and providing, to the user, an indication of the reduced range in which a further dose of tacrolimus is to be taken; and
      transmitting an alert message.

2. A data processing system as claimed in claim 1, wherein the at least one processor is configured to execute a set of computer readable instructions to apply a dose predictor to perform:
   determining a dosage level of Tacrolimus to be taken by the patient by applying an exponential decay function starting from an initial dosage level of Tacrolimus at a start of treatment to determine the dosage level, wherein the decay rate is dependent upon a weight of the patient.

3. A data processing system as claimed in claim 1, wherein the applying the dose predictor to determine the dosage level further comprises applying an update function to an output of the exponential decay function based on a:

   a further predicted concentration of tacrolimus in serum; and
   an earlier output of the update function,
   wherein optionally the applying the dose predictor to determine the dosage level further comprises rounding an output of the update function to one of a set of available dosage amounts for tacrolimus.

4. A data processing system as claimed in any of claims 2 to 3, wherein the at least one processor is configured to execute a set of computer readable instructions to:
   apply the dose predictor to determine a dosage level for each of a predefined number of initial doses of tacrolimus.

5. A data processing system as claimed in claim 4, wherein the at least one processor is configured to execute the set of computer readable instructions to, in response to determining that the predefined number of initial doses of tacrolimus have been administered, perform the steps of:

   calculating the baseline tacrolimus concentration value;
   calculating the deviation of the further measurement; and
   determining that the deviation exceeds a predefined limit.

6. A data processing system as claimed in any preceding claim, wherein the at least one measured tacrolimus concentration in the serum of the patient comprises a plurality of measured Tacrolimus concentration values.

7. A data processing system as claimed in any preceding claim, wherein the at least one processor being configured to execute a set of computer readable instructions to cause the following steps to be performed:

   providing to the user, an indication of a further range around the timing, the further range being smaller than the range; and
   in response to determining that the deviation exceeds the predefined amount, reducing the further range and providing the user an indication of the reduced further range.

8. A data processing system as claimed in any preceding claim, wherein the at least one processor is configured to execute the set of computer readable instructions to cause the following steps to be performed:
   in response to receiving user input indicating a dose timing that falls within the range, causing a first message to be displayed on a display of the data processing system.

9. A data processing system as claimed in any preceding claim, wherein the at least one processor is configured to execute the set of computer readable instructions to check whether the deviation exceeds the predefined limit in response to determining that the baseline tacrolimus concentration value is between an upper bound and a lower bound.

10. A data processing system as claimed in any preceding claim, wherein the at least one processor is configured to execute a set of computer readable instructions to cause the following steps to be performed:

    calculating a deviation in a measured tacrolimus concentration of the at least one measured tacrolimus concentration from an earlier value of the baseline tacrolimus concentration; and
    performing the steps of reducing the range and transmitting an alert message in response to determining that the deviation from the earlier value exceeds a further predefined limit.

11. A data processing system as claimed in any preceding claim, wherein the at least one processor is configured to execute a set of computer readable instructions to cause the following steps to be performed:

    checking that a deviation in measured concentration from an earlier baseline tacrolimus concentration value did not exceed the predefined limit; and
    performing the steps of reducing the range and transmitting an alert message in response to determining that the deviation from the earlier baseline tacrolimus concentration value did not exceed the predefined limit.

12. A data processing system as claimed in any preceding claim, wherein the step of predicting the concentration of tacrolimus in serum of a patient is at least one of:

    performed in dependence upon an indication of the clearance time for a Tac dose for the patient; and
    performed based on predicting a concentration of tacrolimus in serum of the patient is performed based on an earlier predicted tacrolimus concentration value.

13. A data processing system as claimed in any preceding claim, wherein the at least one processor being configured to execute a set of computer readable instructions to cause the following steps to be performed:

    for each of a set of a number of doses tacrolimus, determining a deviation in timing of the respective dose from the timing for repeated administration of tacrolimus to the patient;
    determine for each of the plurality of time periods, an average of the deviations in timing associated with each of a plurality of the doses taken within the respective time period; and
    set the range around the timing at which a dose of tacrolimus is to be taken in dependence upon the lowest of the averages for each of the plurality of time periods.

14. A computer implemented method comprising:

    providing to a user, an indication of a timing for repeated administration of tacrolimus to a patient;
    providing to the user, an indication of a range around the timing at which a dose of tacrolimus is to be taken;
    predicting a concentration of tacrolimus in serum of a patient based on an administered dosage of tacrolimus, a first measured tacrolimus concentration in the serum of the patient, and an indication of a weight of the patient, wherein the predicted concentration corresponds to a time following a measurement time of the first measured tacrolimus concentration;

calculating a baseline tacrolimus concentration value using an average of the predicted concentration of tacrolimus in serum and at least one measured tacrolimus concentration in the serum of the patient;

calculating a deviation of a further measurement of the tacrolimus concentration in serum of the patient from the baseline tacrolimus concentration value; and

in response to determining that the deviation exceeds a predefined limit:

> reducing the range and providing, to the user, an indication of the reduced range in which a further dose of tacrolimus is to be taken; and
>
> transmitting an alert message.

15. A computer program comprising a set of computer readable instructions, which when executed by at least one processor cause a method to be performed, the method comprising:

> providing to a user, an indication of a timing for repeated administration of tacrolimus to a patient;
>
> providing to the user, an indication of a range around the timing at which a dose of tacrolimus is to be taken;
>
> predicting a concentration of tacrolimus in serum of a patient based on an administered dosage of tacrolimus, a first measured tacrolimus concentration in the serum of the patient, and an indication of a weight of the patient, wherein the predicted concentration corresponds to a time following a measurement time of the first measured tacrolimus concentration;
>
> calculating a baseline tacrolimus concentration value using an average of the predicted concentration of tacrolimus in serum and at least one measured tacrolimus concentration in the serum of the patient;
>
> calculating a deviation of a further measurement of the tacrolimus concentration in serum of the patient from the baseline tacrolimus concentration value; and
>
> in response to determining that the deviation exceeds a predefined limit:
>
>> reducing the range and providing, to the user, an indication of the reduced range in which a further dose of tacrolimus is to be taken; and
>>
>> transmitting an alert message.

Figure 1

Figure 2

EP 4 749 635 A1

EP 4 749 635 A1

Figure 3

Figure 4

Figure 5

S610 — Determine Baseline

S620 — Determine Deviation Value

S630 — Is the baseline between bounds?

N → S635 — Alert MD

Y → S640 — Are deviation/s greater than the predefined bounds?

N → S620

Y → S650 — Deviation previously flagged?

Y → S610

N → S660 — Reduce coaching bands

S670 — Alert MD

Figure 6

600

## Table 1

| | DATE | WEIGHT (Kg) | DOSE (Mg) | Predicted PEAK | PREDICTED Tacrolimus Value | Lowest PREDICTED | Highest PREDICTED | ACTUAL |
|---|---|---|---|---|---|---|---|---|
| 1 | 30/07/18 | 44.0 | 2.50 | 113.6 | 20.2 | 17.6 | 24.9 | 20.2 |
| 2 | 01/08/18 | 44.5 | 2.50 | 224.7 | 17.2 | 15.0 | 21.1 | 15.6 |
| 3 | 08/08/18 | 45.5 | 1.50 | 131.9 | 14.5 | 4.6 | 24.3 | 11.9 |
| 4 | 15/08/18 | 46.0 | 1.50 | 130.4 | 10.7 | 7.5 | 14.0 | 11.1 |
| 5 | 22/08/18 | 46.5 | 1.50 | 129.0 | 10.3 | 8.1 | 12.5 | 10.6 |
| 6 | 12/09/18 | 47.5 | 1.00 | 84.2 | 9.8 | 8.2 | 11.5 | 9.9 |
| 7 | 26/09/18 | 49.3 | 1.00 | 81.1 | 8.9 | 7.6 | 10.2 | 9.4 |
| 8 | 10/10/18 | 50.5 | 1.00 | 79.2 | 8.5 | 7.4 | 9.6 | 8.2 |
| 9 | 24/10/18 | 51.0 | 0.75 | 58.82 | 7.4 | 6.5 | 8.3 | 7.9 |
| 10 | 07/11/18 | 52.0 | 0.75 | 57.69 | 7.0 | 6.19 | 7.84 | 7.5 |
| 11 | 28/11/18 | 53.0 | 0.75 | 56.60 | 6.7 | 5.94 | 7.40 | 7.4 |
| 12 | 19/12/18 | 53.5 | 0.75 | 56.07 | 6.6 | 5.93 | 7.31 | 5.9 |
| 13 | 08/01/19 | 56.0 | 0.75 | 53.57 | 5.3 | 4.64 | 5.91 | 8.1 |
| 14 | 05/02/19 | 56.8 | 0.75 | 52.82 | 7.4 | 6.61 | 8.14 | 7.4 |
| 15 | 26/02/19 | 57.2 | 0.75 | 52.45 | 6.5 | 5.79 | 7.20 | 6.5 |
| 16 | | | | | 5.8 | 5.14 | 6.43 | |

Figure 7A

Table 2

| Clearance Time | Scaler |
|---|---|
| 10.0 hrs | 0.85 |
| 10.5 hrs | 0.90 |
| 11.0 hrs | 0.95 |
| 11.5 hrs | 1.00 |
| 12.0 hrs | 1.05 |
| 12.5 hrs | 1.10 |
| 13.0 hrs | 1.15 |

Figure 7B

Figure 8

Figure 9

Table 4

| Measurement | Ideal Tac Dose (mg) |
|---|---|
| 1 | 2.50 |
| 2 | 1.91 |
| 3 | 1.65 |
| 4 | 1.40 |
| 5 | 1.22 |
| 6 | 1.09 |
| 7 | 1.00 |
| 8 | 0.94 |
| 9 | 0.89 |
| 10 | 0.86 |
| 11 | 0.84 |
| 12 | 0.82 |
| 13 | 0.81 |
| 14 | 0.80 |
| 15 | 0.79 |

Table 3

| Weight | Offset | Decay Rate |
|---|---|---|
| < 60 kg | 6.3 | 0.34 |
| 60 – 80 kg | 6.8 | 0.44 |
| 80 – 100 kg | 7.3 | 0.47 |
| > 100 kg | 7.8 | 0.49 |

Figure 10

Table 6

| Measurement | Dose Used (mg) |
|---|---|
| 1 | 2.50 |
| 2 | 2.25 |
| 3 | 1.75 |
| 4 | 1.50 |
| 5 | 1.25 |
| 6 | 1.25 |
| 7 | 1.00 |
| 8 | 1.00 |
| 9 | 1.00 |
| 10 | 1.00 |
| 11 | 1.00 |
| 12 | 0.75 |
| 13 | 0.75 |
| 14 | 0.75 |
| 15 | 0.75 |

Table 5

| Measurement | Bayesian Update (mg) |
|---|---|
| 1 | 2.50 |
| 2 | 2.22 |
| 3 | 1.70 |
| 4 | 1.47 |
| 5 | 1.23 |
| 6 | 1.09 |
| 7 | 0.98 |
| 8 | 0.90 |
| 9 | 0.84 |
| 10 | 0.80 |
| 11 | 0.77 |
| 12 | 0.75 |
| 13 | 0.73 |
| 14 | 0.70 |
| 15 | 0.73 |

Figure 11

```
                    ┌─────────────────────────────┐
                    │         Tac predictor       │
                    └─────────────────────────────┘
                                   │
                      ┌────────────┴────────────┐
                      ▼                         ▼
        ┌──────────────────────┐    ┌──────────────────────┐
        │   Patient Coaching   │    │    Patient Dose      │
        │                      │    │     Predictor        │
        └──────────────────────┘    └──────────────────────┘
                  │                            │
                  ▼                            ▼
        ┌──────────────────────┐    ┌──────────────────────┐
        │  Tac levels used to  │    │  Tac levels used in  │
        │  establish baseline  │    │  Bayesian dose       │
        │                      │    │  predictor           │
        └──────────────────────┘    └──────────────────────┘
                  │                            │
                  ▼                            ▼
        ┌──────────────────────┐    ┌──────────────────────┐
        │   Coaching band      │    │   New dose values    │
        │   elasticity varied as│   │   generated          │
        │   Tac value deviates │    │   automatically      │
        └──────────────────────┘    └──────────────────────┘
```

Figure 12

EP 4 749 635 A1

S1320

```
         ┌──────────┐
         │  1ˢᵗ M?  │──Y──▶┌────────────────────────────────────┐      ┌────────────────────────────┐
         │          │      │ Calculate PP as Dose*100/(weight*5%)│─────▶│  Determine clearance time  │───── S1340
         └──────────┘      └────────────────────────────────────┘      └────────────────────────────┘
             │                                                                        │
S1310       N                                                                         ▼
             │              ┌────────────────────────────────────┐      ┌────────────────────────────┐
             └─────────────▶│ Calculate PP as Dose*200/(weight*5%)│      │    Convert to scalar value │───── S1350
                            └────────────────────────────────────┘      └────────────────────────────┘
                                              │                                       │
                                           S1350                                      │
                                              │                                       │
                                              ▼                                       ▼
                            ┌──────────────────────────────────────────────────┐
                            │          Obtain measured actual Tac value         │───── S1360
                            └──────────────────────────────────────────────────┘
                                              │
                                              ▼
                                    ┌──────────────┐
                                    │     1ˢᵗ      │──Y──▶┌──────────────────────────────────────────┐
                                    │  prediction  │      │ Set predicted value equal to the actual Tac Value │───── S1380
                                    │      ?       │      └──────────────────────────────────────────┘
                                    └──────────────┘
                                        │
                                     S1370   N  ┌──────────────────────────────────────────────────┐
                                        └──────▶│ Calculate new prediction as PP*ATV*Scalar/(PP+Previous │───── S1390
                                                │                    prediction)                     │
                                                └──────────────────────────────────────────────────┘
1300
```

Figure 13

Figure 14

EP 4 749 635 A1

Figure 15

```
  ┌─────────────────────────────────┐
  │ Calculate deviation of dose      │
  │ timing from optimal timing       │───── S1610
  └─────────────────────────────────┘
              │
              ▼
           ◇ Current
        N  time period
  ───◇   complete?  ◇───── S1620
              │
              │ Y
              ▼
  ┌─────────────────────────────────┐
  │ Calculate average deviation for  │
  │ the time period                  │
  └─────────────────────────────────┘
         S1630
```

Update Best_time and reduce coaching band width — S1650

New lowest average deviation? — S1640

1600

Figure 16

Europäisches
Patentamt
European
Patent Office
Office européen
des brevets

## EUROPEAN SEARCH REPORT

**Application Number**

EP 24 21 4924

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 113 930 495 B (SHANGHAI GENERAL HOSPITAL) 28 March 2023 (2023-03-28) * paragraphs [0045], [0078] - [0081] * ----- | 1-15 | INV. G16H20/10 |
| A | CN 117 126 932 A (CHINA JAPAN FRIENDSHIP HOSPITAL CHINA JAPAN FRIENDSHIP INST OF CLINICA) 28 November 2023 (2023-11-28) * claim 1 * ----- | 1-15 | |
| E | GB 2 630 386 A (HEALTH AI HOLDINGS LTD [GB]) 27 November 2024 (2024-11-27) * the whole document * ----- | 1-15 | |
| | | | **TECHNICAL FIELDS SEARCHED (IPC)** |
| | | | G16H |

The present search report has been drawn up for all claims

| Place of search | Date of completion of the search | Examiner |
|---|---|---|
| Munich | 29 April 2025 | Reinbold, Bernhard |

EPO FORM 1503 03.82 (P04C01)

## ANNEX TO THE EUROPEAN SEARCH REPORT
## ON EUROPEAN PATENT APPLICATION NO.

EP 24 21 4924

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2025

| Patent document cited in search report | | Publication date | Patent family member(s) | Publication date |
|---|---|---|---|---|
| CN 113930495 | B | 28-03-2023 | NONE | |
| CN 117126932 | A | 28-11-2023 | NONE | |
| GB 2630386 | A | 27-11-2024 | NONE | |

EPO FORM P0459

For more details about this annex : see Official Journal of the European Patent Office, No. 12/82